(19) Europäisches Patentamt
European Patent Office
Office européen des brevets

(11) **EP 4 406 481 A2**

(12) **EUROPEAN PATENT APPLICATION**
published in accordance with Art. 153(4) EPC

(43) Date of publication:
**31.07.2024 Bulletin 2024/31**

(21) Application number: **22873175.8**

(22) Date of filing: **21.09.2022**

(51) International Patent Classification (IPC):
*A61B 5/349* (2021.01)    *A61B 5/00* (2006.01)
*G16H 50/20* (2018.01)    *G16H 50/50* (2018.01)
*G16H 50/70* (2018.01)    *G06N 3/08* (2023.01)
*G06N 3/04* (2023.01)

(52) Cooperative Patent Classification (CPC):
**A61B 5/00; A61B 5/349; G06N 3/04; G06N 3/08;
G16H 50/20; G16H 50/50; G16H 50/70**

(86) International application number:
**PCT/KR2022/014131**

(87) International publication number:
**WO 2023/048470 (30.03.2023 Gazette 2023/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.09.2021 KR 20210126786
20.09.2022 KR 20220118351**

(71) Applicant: **Medical AI Co., Ltd.
Seoul 06302 (KR)**

(72) Inventors:
• **KWON, Joonmyoung
Seoul 06302 (KR)**
• **JANG, Jonghwan
Seoul 06302 (KR)**

(74) Representative: **Patentanwaltskanzlei
Matschnig & Forsthuber OG
Biberstraße 22
Postfach 36
1010 Wien (AT)**

(54) **MEDICAL DATA ANALYSIS METHOD BASED ON EXPLAINABLE ARTIFICIAL INTELLIGENCE, PROGRAM, AND DEVICE**

(57) According to an embodiment of the present disclosure, there are disclosed a method, program and device for interpreting medical data based on explainable artificial intelligence, which are performed by a computing device. The method may include: training a first neural network model to estimate a positive or negative for a disease with respect to first medical data based on the first medical data; and training a second neural network model configured to transform features of second medical data so that a positive or negative for the disease with respect to the second medical data is estimated to be opposite by using the trained first neural network model.

FIG. 3

## Description

### Technical Field

The present disclosure relates to deep learning technology in the medical field, and more particularly to a method of interpreting the decision-making process of a deep learning model used for the detection of disease and explaining the correlations between medical data and disease based on the results of the interpretation.

### Background Art

The biggest limitation of artificial intelligence is that it cannot reveal the accurate limits of a decision-making process, i.e., a black box. In other words, we cannot know the process and reasons related to the erroneous determination of artificial intelligence. This limitation is critical in the medical field because it hinders the monitoring and detection of errors. Many researches are currently being attempted to overcome this limitation.

For example, one of the existing researches uses a sensitivity map to mark data regions that are important for the detection of disease. However, the sensitivity map has the disadvantage of only displaying regions deemed important and not explaining features of the regions that are related to disease. Another of the existing researches uses an ensemble technique that detects disease through the integration of modules that describe respective features of the disease. The corresponding research can explain features that were involved in the decision to detect disease. However, the corresponding research uses only the features predetermined by the modules, so that there is a limitation in that it is difficult to know what kind of decision making artificial intelligence actually performs.

### Disclosure

### Technical Problem

The present disclosure has been conceived in response to the above-described background technology, and an object of the present disclosure is to provide a method that can explain the reasons for the determination and decision of a deep learning model for the detection of disease and interpret the features of medical data that are related to a disease based on this explanation.

However, the objects to be accomplished by the present disclosure are not limited to the object mentioned above, and other objects not mentioned may be clearly understood based on the following description.

### Technical Solution

According to an embodiment of the present disclosure for accomplishing the above-described object, there is disclosed a method of interpreting medical data based on explainable artificial intelligence, which is performed by a computing device. The method may include: training a first neural network model to estimate a positive or negative for a disease with respect to first medical data based on the first medical data; and training a second neural network model configured to transform features of second medical data so that a positive or negative for the disease with respect to the second medical data is estimated to be opposite by using the trained first neural network model.

Alternatively, the second neural network model may include: a first sub-neural network model configured to generate third medical data by transforming features of the second medical data so that a positive or negative for the disease is estimated to be opposite to a result with respect to the second medical data; and a second sub-neural network model configured to generate fourth medical data by transforming features of the third medical data so that a positive or negative for the disease is estimated to be the same as a result with respect to the second medical data.

Alternatively, training the second neural network model configured to transform the one or more features of the second medical data so that the positive or negative for the disease with respect to the second medical data is estimated to be opposite by using the trained first neural network model may include: transforming features of the second medical data by using the second neural network model, and estimating a positive or negative with respect to the second medical data whose features have been transformed by using the trained first neural network model; and training the second neural network model based on a loss function that uses at least one of the second medical data whose features have been transformed and a positive or negative estimation result with respect to the second medical data whose features have been transformed as an input variable.

Alternatively, transforming the features of the second medical data by using the second neural network model and estimating the positive or negative with respect to the second medical data whose features have been transformed by using the trained first neural network model may include: generating third medical data estimated to be positive, which is opposite to a result with respect to the second medical data by transforming features of the second medical data

estimated to be negative based on a first sub-neural network model included in the second neural network model; and generating fourth medical data corresponding to the second medical data estimated to be negative by transforming features of the third medical data based on a second sub-neural network model included in the second neural network model.

Alternatively, transforming the features of the second medical data by using the second neural network model and estimating the positive or negative with respect to the second medical data whose features have been transformed by using the trained first neural network model may further include: generating fifth medical data corresponding to the second medical data estimated to be negative by inputting the second medical data estimated to be negative to the second sub-neural network model; and generating prediction data indicative of a positive or negative estimation result for the disease with respect to the third medical data by inputting the third medical data to the trained first neural network model.

Alternatively, the loss function may include: a first loss function for evaluating whether the first sub-neural network model generates an output corresponding to a positive for the disease by transforming features of an input corresponding to a negative for the disease; a second loss function for evaluating whether the second sub-neural network model restores an input of the first sub-neural network model by transforming features of the output of the first sub-neural network model; and a third loss function for evaluating whether the second sub-neural network model generates an output, which is the same as the input of the first sub-neural network model, based on the input of the first sub-neural network model.

Alternatively, training the second neural network model based on the loss function that uses the at least one of the second medical data whose features have been transformed and the positive or negative estimation result with respect to the second medical data whose features have been transformed as the input variable may include training the first sub-neural network model and the second sub-neural network model based on a loss function that uses at least one of the fourth medical data, the fifth medical data, and the prediction data as an input variable.

Alternatively, training the first sub-neural network model and the second sub-neural network model based on the loss function that uses the at least one of the fourth medical data, the fifth medical data, or the prediction data as the input variable may include: performing the operation of the first loss function using the prediction data as an input variable, and training the first sub-neural network model so that a first loss that is a result of the operation of the first loss function is reduced; performing the operation of the second loss function using the fourth medical data as an input variable, and training at least one of the first sub-neural network model or the second sub-neural network model so that the second loss that is a result of the operation of the second loss function is reduced; and performing the operation of the third loss function using the fifth medical data as an input variable, and training the second sub-neural network model so that a third loss that is a result of the operation of the third loss function is reduced.

Alternatively, the first medical data and the second medical data may include electrocardiogram data. Furthermore, the disease may include hyperkalemia.

Alternatively, the features of the second medical data may be based on the morphological differences between the electrocardiogram data negative for hyperkalemia and the electrocardiogram data positive for hyperkalemia.

Meanwhile, according to alternative embodiment of the present disclosure for accomplishing the above-described object, there is disclosed a method of interpreting medical data based on explainable artificial intelligence, which is performed by a computing device. The method includes: acquiring medical data including electrocardiogram data; and generating output data, with respect to which a positive or negative for a disease is estimated to be opposite to a result with respect to the medical data by a first neural network model, by transforming features of the medical data based on a second neural network model. In this case, the second neural network model may have been pre-trained using the first neural network model trained to estimate a positive or negative for the disease with respect to medical data.

Alternatively, the method may further include generating a user interface for visually comparing the medical data and the output data.

According to an embodiment of the present disclosure for accomplishing the above-described object, there is disclosed a computer program stored in a computer-readable storage medium. The computer program performs operations for interpreting medical data based on explainable artificial intelligence when executed on one or more processors. In this case, the operations may include operations of: training a first neural network model to estimate a positive or negative for a disease with respect to first medical data based on the first medical data; and training a second neural network model configured to transform features of second medical data so that a positive or negative for the disease with respect to the second medical data is estimated to be opposite by using the trained first neural network model.

According to an embodiment of the present disclosure for accomplishing the above-described object, there is disclosed a computing device for interpreting medical data based on explainable artificial intelligence. The computing device may include: a processor including at least one core; memory including program codes that are executable on the processor; and a network unit configured to acquire medical data. The processor may train a first neural network model to estimate a positive or negative for a disease with respect to first medical data based on the first medical data, and may train a second neural network model configured to transform features of second medical data so that a positive or negative for the disease with respect to the second medical data is estimated to be opposite by using the trained first neural network model.

**Advantageous Effects**

The present disclosure may provide the method that can explain the reasons for the determination and decision of a deep learning model for the detection of disease and interpret the features of medical data that are related to a disease based on this explanation.

**Description of Drawings**

FIG. 1 is a block diagram of a computing device according to an embodiment of the present disclosure;
FIG. 2 is a block diagram showing a process of classifying medical data as positive or negative for a disease by using a first neural network model according to an embodiment of the present disclosure;
FIG. 3 is a block diagram showing a process of interpreting medical data by using a second neural network model according to an embodiment of the present disclosure;
FIG. 4 is a graph showing simulation results of a second neural network model trained according to an embodiment of the present disclosure;
FIG. 5 is a flowchart showing a method of training a neural network model according to an embodiment of the present disclosure; and
FIG. 6 is a flowchart showing a method of interpreting medical data by using a neural network model according to an embodiment of the present disclosure.

**Mode for Invention**

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "acquisition" used herein can be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The term "block" used herein may be understood as a set of components classified based on various criteria such as type, function, etc. Accordingly, the components classified as each "block" may be changed in various manners depending on the criteria. For example, a neural network "block" may be understood as a set of neural networks including one or more neural networks. In this case, it can be assumed that the neural networks included in the neural network "block" perform predetermined operations to achieve a common purpose that serves as a classification criterion.

The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

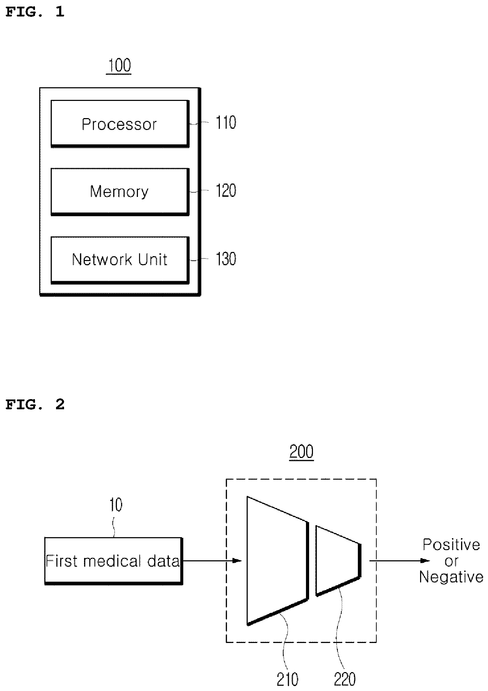

FIG. 1 is a block diagram of a computing device according to an embodiment of the present disclosure.

A computing device 100 according to an embodiment of the present disclosure may be a hardware device or part of a hardware device that performs the comprehensive processing and calculation of data, or may be a software-based computing environment that is connected to a communication network. For example, the computing device 100 may be a server that performs an intensive data processing function and shares resources, or may be a client that shares resources through interaction with a server. Furthermore, the computing device 100 may be a cloud system in which a plurality of servers and clients interact with each other and comprehensively process data. Since the above descriptions are only examples related to the type of computing device 100, the type of computing device 100 may be configured in various manners within a range understandable to those skilled in the art based on the content of the present disclosure.

Referring to FIG. 1, the computing device 100 according to an embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to an embodiment of the present disclosure may be understood as a configuration unit including hardware and/or software for performing computing operation. For example, the processor 110 may read a computer program and perform data processing for machine learning. The processor 110 may process computational processes such as the processing of input data for machine learning, the extraction of features for machine learning, and the calculation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 may train a deep learning model, used to interpret the determination and decision-making process of artificial intelligence for the detection of disease, based on medical data. The processor 110 may train a deep learning model based on medical data so that the deep learning model provides grounds for the determination of artificial intelligence on the features of medical data that affect the occurrence of a specific disease. For example, the processor 110 may transform medical data so that the presence/absence of a disease (i.e., a positive for the disease/a negative for the disease) is predicted to be opposite by inputting the medical data to the deep learning model. When the medical data is the medical data of a person who has developed a disease (i.e. a person who has tested positive), the processor 110 may transform the input medical data to be predicted to be the medical data of a person who does not have the disease (i.e. a person who has tested negative) by using a deep learning model. When the medical data is the medical data of a person who has not developed the disease, the processor 110 may transform the input medical data to be predicted to be the medical data of a person who has the disease by using a deep learning model. The data generated through this transformation may exhibit features opposite to those of input based on the presence/absence of a disease. The processor 110 may train a model by repeatedly performing the forward propagation and backpropagation of the deep learning model so that the features opposite to those of the input represented by the medical data transformed by the deep learning model are clearly represented. The processor 110 may generate grounds for explaining the decision-making and determination process of artificial intelligence for the diagnosis of a specific disease by using the deep learning model trained through the above process.

The processor 110 may generate a user interface for the interpretation of the determination and decision-making process of artificial intelligence for the detection of disease. The processor 110 may generate a user interface for the visualization of the grounds for the determination of artificial intelligence provided using the deep learning model trained as described above. For example, the processor 110 may generate a graphics-based user interface so that the medical data transformed by the trained deep learning model and the medical data before the transformation corresponding to the input of the deep learning model can be compared with each other. The medical data transformed by the deep learning model may exhibit features opposite to those of the input based on the presence/absence of a disease. Accordingly, when the medical data transformed by the deep learning model and the medical data before the transformation are compared with each other, we may easily determine the features of medical data that affect the occurrence of the disease. Additionally, when the medical data transformed by the deep learning model and the medical data before the transformation are compared with each other, we may easily determine how artificial intelligence for the detection of disease interprets the features of medical data that affect the occurrence of the disease. In order to enable a user to easily perform such comparison and determination, the processor 110 may generate a user interface for graphically representing the medical data transformed by the deep learning model and the medical data before the transformation.

The memory 120 according to an embodiment of the present disclosure may be understood as a configuration unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage data necessary for the processor 110 to perform computation, the combination of data, and program codes executable on the processor 110. For example, the memory 120 may store medical data received through the network unit 130, which will be described later. The memory 120 may store program codes configured to operate the neural network model to receive medical data and perform learning, program codes configured to operate the neural network model to receive medical data and perform inference in accordance with the purpose of use of the computing device 100, processed data generated as program codes are executed, etc.

The network unit 130 according to an embodiment of the present disclosure may be understood as a configuration unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wideband wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data necessary for the processor 110 to perform computation through wired/wireless communication with any system or client or the like. Furthermore, the network unit 130 may transmit data generated through the computation of the processor 110 through wired/wireless communication with any system or client or the like. For example, the network unit 130 may receive medical data through communication with a database within a hospital environment, a cloud server configured to perform tasks such as the standardization of medical data, a computing device, or the like. The network unit 130 may transmit the output data of the neural network model, intermediate data and processed data acquired from the computation process of the processor 110, etc. through communication with the above-described database, server, or computing device.

FIG. 2 is a block diagram showing a process of classifying medical data as positive or negative for a disease by using a first neural network model according to an embodiment of the present disclosure.

Referring to FIG. 2, the processor 110 of the computing device 100 according to an embodiment of the present disclosure may train a first neural network model 200 for the detection of disease based on first medical data 10. The processor 110 may train the first neural network model 200 based on the medical data 10 so that the first neural network model 200 classifies medical data as positive or negative for a disease. In this case, the first medical data 10 may include biosignal-related data for which morphological interpretation is used to diagnose a disease.

For example, the processor 110 may predict the subject of measurement of the first medical data 10 to be positive or negative for hyperkalemia by inputting electrocardiogram data, included in the first medical data 10, to the first neural network model 200. The processor 110 may compare a positive or negative prediction result for hyperkalemia with respect to the first medical data 10 with GT (ground truth) by using a loss function. The processor 110 may reconstruct parameters, such as weight, of the first neural network model 200 based on the comparison result. The processor 110 may train the first neural network model 200 based on the first medical data 10 by repeatedly performing the above operation until the loss function converges. The training of the first neural network model 200 may be performed based on semi-supervised learning, unsupervised learning, reinforcement learning, etc. in addition to supervised learning, which is the example described above.

Meanwhile, the first neural network model 200 may include a neural network 210 for extracting features from the first medical data 10 and a neural network 220 for classifying the data as positive or negative for a disease based on the extracted features. For example, the neural network 210 for the feature extraction of the first neural network model 200 may include a residual neural network. The neural network 210 for feature extraction may be composed of n residual blocks (n is a natural number). Each of the n residual blocks may include a convolution layer, an activation layer, a dropout layer, and a batch normalization layer. The neural network 220 for the classification of the first neural network model 200 may average the feature map, generated by the neural network 210 for feature extraction, on a channel-wise basis. The neural network 220 for classification may calculate the probability of being positive or negative for the disease through a fully connected layer (FCL) and a softmax activation layer.

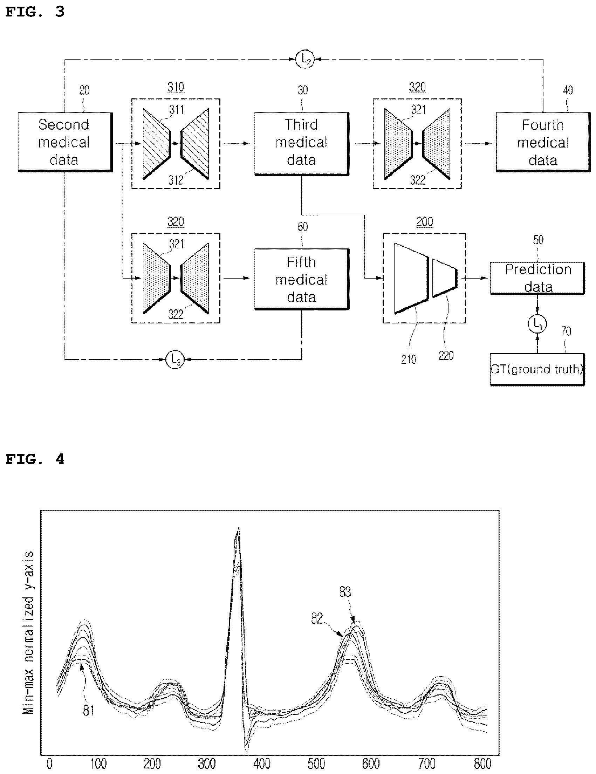

FIG. 3 is a block diagram showing a process of interpreting medical data by using a second neural network model according to an embodiment of the present disclosure.

The processor 110 of the computing device 100 according to an embodiment of the present disclosure may train the second neural network model for the interpretation of the determination and decision-making process of the first neural network model 200 for the detection of disease based on second medical data 20. The processor 110 may train a second neural network model based on the second medical data 20 so that the second neural network model generates a counter factor for the second medical data 20 based on the task of the first neural network model 200. The processor 110 may train the second neural network model so that the second neural network model generates comparison data that can be used to explain the data interpretation and decision-making process of the first neural network model 200. In this case, the processor 110 may use the pre-trained first neural network model 200 in the process of training the second neural network model. Additionally, the second medical data 20 may include biosignal-related data for which morphological interpretation is used to diagnose disease.

More specifically, referring to FIG. 3, the processor 110 may transform features of the second medical data 20 based on the first sub-neural network model 310 included in the second neural network model. In this case, the feature transformation of the second medical data 20 may be understood as an operation process of generating new data based on the second medical data 20 so that a result opposite to the prediction result of the first neural network model 200 for the second medical data 20 is obtained. The processor 110 may generate third medical data 30 by transforming features of the second medical data 20 through the first sub-neural network model 310 so that a positive or negative for the disease is estimated to be opposite to that for the second medical data 20. In this case, the first sub-neural network model 310 may include a neural network 311 for extracting features from the second medical data 20 and a neural network 312 for generating the third medical data 30 with respect to which the positive or negative for the disease is estimated to be opposite to that for the second medical data 20 by transforming the extracted features. The neural network 311 for the feature extraction of the first sub-neural network model 310 may correspond to the neural network 210 for the feature extraction of the first neural network model 200. Accordingly, in the process of training the first sub-neural network model 310, the training of the neural network 311 for feature extraction may not be performed, but only the training of the neural network 312 that generates the third medical data 30 may be performed.

For example, the processor 110 may input the electrocardiogram data, included in the second medical data 20, to the first sub-neural network model 310. In this case, the electrocardiogram data included in the second medical data 20 may be electrocardiogram data of a person who is negative for hyperkalemia. The processor 110 may transform morphological features, such as the PR interval, QRS duration and T amplitude, of electrocardiogram data, included in the second medical data 20, through the first sub-neural network model 310. Furthermore, the processor 110 may generate the third medical data 30, including electrocardiogram data estimated to be positive for hyperkalemia, through the transformation of morphological features of the second medical data 20 based on the first sub-neural network model 310. That is, the processor 110 may generate electrocardiogram data, estimated to be positive for hyperkalemia, using the first neural network model 200 by transforming morphological features of electrocardiogram data, negative for hyperkalemia, using the first sub-neural network model 310.

The processor 110 may transform features of the third medical data 30 based on the second sub-neural network model 320 included in the second neural network model. In this case, the feature transformation of the third medical data 30 may be understood as an operation process of restoring the third medical data 30 to the second medical data 20 so that the same result is obtained as the prediction of the first neural network model 200 for the second medical data 20. The processor 110 may generate fourth medical data 40 corresponding to the second medical data 20 through the second sub-neural network model 320 by transforming features of the third medical data 30 so that a positive or negative for the disease is estimated to be the same as a result for the second medical data 20. In this case, the second sub-neural network model 320 may include a neural network 321 for extracting features from the third medical data 30, and a neural network 322 for generating the fourth medical data 40 corresponding to the second medical data 20 by transforming the extracted features. The neural network 321 for the feature extraction of the second sub-neural network model 320 may correspond to the neural network 210 for the feature extraction of the first neural network model 200. Accordingly, in the process of training the second sub-neural network model 320, the training of the neural network 321 for feature extraction may not be performed, but only the training of the neural network 322 that generates the fourth medical data 40 may be performed.

For example, the processor 110 may input the third medical data 30, including the electrocardiogram data generated through the first sub-neural network model 310, to the second sub-neural network model 320. In this case, the electrocardiogram data included in the third medical data 30 may be data that is estimated to be positive for hyperkalemia by the first neural network model 200. The processor 110 may transform morphological features of the electrocardiogram data, included in the third medical data 30, based on indices, such as the PR interval, QRS duration and T amplitude, of the electrocardiogram through the second sub-neural network model 320. Furthermore, the processor 110 may generate the fourth medical data 40 close to the second medical data 20 through the transformation of morphological features of the third medical data 30 based on the second sub-neural network model 320. In other words, the processor 110 may perform an operation of restoring the third medical data 30, generated by the first sub-neural network model 310, back to the second medical data 20 through the second sub-neural network model 320. That is, the processor 110 may generate electrocardiogram negative for hyperkalemia by transforming morphological features of electrocardiogram data, estimated to be positive for hyperkalemia, using the second sub-neural network model 320.

Based on the above description, the processor 110 may transform the domain of the second medical data 20 by using the first sub-neural network model 310 and the second sub-neural network model 320. In this case, the domain of the second medical data 20 may be understood as a domain that is classified based on whether it is positive or negative for the disease. For example, the processor 110 may transform the second medical data 20 corresponding to the negative domain of a specific disease into a positive domain through the first sub-neural network model 310. The processor 110 may transform the second medical data 20, which has been transformed into the positive domain through the second sub-neural network model 320, back into the negative domain. In this case, the transformation into the positive domain using the first sub-neural network model 310 and the transformation into the negative domain using the second sub-neural network model 320 may be understood as an operation process of transforming the style of the second medical data 20 while maintaining the content of the second medical data 20 as much as possible.

The processor 110 may predict a positive or negative for a disease with respect to the third medical data 30 generated by the first sub-neural network model 310 based on the pre-trained first neural network model 200. The processor 110 may generate prediction data 50 including a positive or negative estimation result for the disease with respect to the third medical data 30 by inputting the third medical data 30 to the pre-trained first neural network model 200. The processor 110 may determine whether the third medical data 30 generated by the first sub-neural network model 310 through the pre-trained first neural network model 200 is predicted to be positive or negative for the disease, contrary to the second medical data 20. That is, the processor 110 may evaluate the operation result of the first sub-neural network model 310 by using the pre-trained first neural network model 200. In other words, the pre-trained first neural network model 200 may be understood as a model for determining the operation result of the first sub-neural network model 310.

For example, the processor 110 may input the third medical data 30, generated by inputting the second medical data 20 including electrocardiogram data negative for hyperkalemia to the first sub-neural network model 310, to the neural network model 200. The processor 110 may extract features of the electrocardiogram data included in the third medical data 30 through the feature extraction neural network 210 of the first neural network model 200. Furthermore, the processor 110 may generate the prediction data 50 including the probability of a positive or negative for hyperkalemia with respect to the third medical data 30 based on features of the third medical data 30 through the classification neural network 220 of the first neural network model 200. When the first sub-neural network model 310 appropriately generates the third medical data 30 in accordance with its task, the first neural network model 200 may output the prediction data 50 including a result in which the third medical data 30 is estimated to be positive for hyperkalemia. That is, the processor 110 may evaluate whether the first sub-neural network model 310 has appropriately generated the third medical data 30, which is positive for hyperkalemia, contrary to the second medical data 20, by transforming features of the second medical data 20 through the first neural network model 200.

The processor 110 may generate fifth medical data 60 corresponding to the second medical data 20 by inputting the second medical data 20 to the second sub-neural network model 320. In this case, the generation of the fifth medical data 60 may be understood as an operation process of evaluating whether the second sub-neural network model 320 has generated data that corresponds to the second medical data 20 in terms of a positive or negative for the disease. The second sub-neural network model 320 may basically perform the task of restoring the second medical data 20 based on the third medical data 30. Accordingly, when the second medical data 20 is input to the second sub-neural network model 320, the second sub-neural network model 320 theoretically needs to generate the fifth medical data 60 corresponding to the second medical data 20. That is, the processor 110 may determine whether the second sub-neural network model 320 appropriately performs its task by inputting the second medical data 20 to the second sub-neural network model 320.

For example, the processor 110 may input second medical data 20, including electrocardiogram data negative for hyperkalemia, to the second sub-neural network model 320. The processor 110 may generate the fifth medical data 60 by transforming morphological features of the electrocardiogram data, included in the second medical data 20, through the second sub-neural network model 320. Since the second sub-neural network model 320 aims to generate data corresponding to the same domain as the second medical data 20 by transforming the style while maintaining the content of the input, the fifth medical data 60 may be the same data as the second medical data 20 that is negative for hyperkalemia. That is, when the second sub-neural network model 320 appropriately generates the fifth medical data 60 in accordance with its task, the second sub-neural network model 320 may be understood as outputting the second medical data 20, negative for hyperkalemia, without change.

Referring to FIG. 3, the processor 110 may train the first sub-neural network model 310 based on a first loss function. In this case, the first loss function may be a loss function for evaluating whether the first sub-neural network model 310 generates an output that is positive or negative for the disease, contrary to an input, by transforming features of the input. Furthermore, the first loss function may use prediction data 50, including a positive or negative estimation result for the disease with respect to the third medical data 30, and ground truth (GT) as input variables.

For example, the first loss function may be represented by Equation 1 below:

$$L_1 = CrossEntropy(y_A, \hat{y}_A) \qquad (1)$$

where $y_A$ may be the ground truth (GT) and $\hat{y}_A$ may be the predicted probability of being positive or negative included in the prediction data 50. Although the cross entropy function is described as the loss function in Equation 1, the present disclosure is not limited to the corresponding function.

The processor 110 may obtain a first loss by performing the operation of the first loss function using the prediction data 50 and the ground truth (GT) as input variables. The processor 110 may train the first sub-neural network model 310 so that the first loss is reduced. That is, the processor 110 may reconstruct parameters of the first sub-neural network model 310 to lower the first loss in order for the first sub-neural network model 310 to deceive the first neural network model 200. For example, when the first sub-neural network model 310 receives electrocardiogram data negative for hyperkalemia, the processor 110 may reconstruct parameters of the generative neural network 312 of the first sub-neural network model 310 in a direction in which the first loss is reduced by repeatedly performing the operation of the first loss function. In this case, the reconstruction of parameters of the generative neural network 312 of the first sub-neural network model 310 may be understood as a process of training the generative neural network 312 of the first sub-neural network model 310 so that the first neural network model 200 predicts the output of the first sub-neural network model 310 to be electrocardiogram data positive for hyperkalemia. In this case, since the feature extraction neural network 311 of the first sub-neural network model 310 corresponds to the feature extraction neural network 210 of the first neural network model 200, the training of the feature extraction neural network 311 of the first sub-neural network model 310 may not be performed in the training process using the first loss function.

The processor 110 may train at least one of the first sub-neural network model 310 or the second sub-neural network model 320 based on a second loss function. In this case, the second loss function is a loss function for evaluating whether the second sub-neural network model 320 restores the input of the first sub-neural network model 310 by transforming features of the output of the first sub-neural network model 310. Furthermore, the second loss function may use the second medical data 20, which is an input to the first sub-neural network model 310, and the fourth medical data 40, which is generated by the second sub-neural network model 320 based on the third medical data 30, as input variables. That is, the second loss function may be a function for comparing the input of the first sub-neural network model 310 and the output of the second sub-neural network model 320 based on the output of the first sub-neural network model 310. For example, the second loss function may be represented by Equation 2 below:

$$L_2 = Mean\ squared\ error(x_{real}{}^A, x_{recover}{}^A) \qquad (2)$$

where $x_{reat}^A$ may be the second medical data 20 and $x_{recover}^A$ may be the fourth medical data 40. Although the mean squared error function is described as the loss function in Equation 2, the present disclosure is not limited to the corresponding function.

The processor 110 may obtain a second loss by performing the operation of the second loss function using the second medical data 20 and the fourth medical data 40 as input variables. The processor 110 may train at least one of the first sub-neural network model 310 or the second sub-neural network model 320 so that the second loss is reduced. That is, the processor 110 may reconstruct parameters of at least one of the first sub-neural network model 310 or the second sub-neural network model 320 to lower the second loss so that the fourth medical data 40 can be restored to the second medical data 20. For example, when the first sub-neural network model 310 receives electrocardiogram data negative for hyperkalemia, the processor 110 may reconstruct parameters of the generative neural network 312 of the first sub-neural network model 310 and the generative neural network 322 of the second sub-neural network model 320 in the direction in which the second loss is reduced by repeatedly performing the operation of the second loss function. In this case, the reconstruction of parameters of the generative neural networks 312 and 322 may be understood as a process of training the generative neural networks 312 and 322 so that the second sub-neural network model 320 generates electrocardiogram data that is negative for hyperkalemia in the same manner as the input of the first sub-neural network model 310. In this case, since the feature extraction neural network 311 of the first sub-neural network model 310 and the feature extraction neural network 321 of the second sub-neural network model 320 correspond to the feature extraction neural network 210 of the first neural network model 200, the training of the feature extraction neural networks 311 and 321 may not be performed in the training process using the first loss function. The processor 110 may prevent the output of the second sub-neural network model 320, which is unrelated to the input of the first sub-neural network model 310, from being generated through the training via the second loss function. In other words, the processor 110 may allow the output of the second sub-neural network model 320 to have a form, in which only the style is transformed while the content of the input of the first sub-neural network model 310 is maintained as much as possible, through the training via the second loss function.

The processor 110 may train the second sub-neural network model 320 based on a third loss function. In this case, the third loss function may be a loss function for evaluating whether the second sub-neural network model 320 generates an output, which is the same as the input of the first sub-neural network model 310, based on the input of the first sub-neural network model 310. Furthermore, the third loss function may use the second medical data 20, which is the input of the first sub-neural network model 310, and the fifth loss function 60, which is generated by the second sub-neural network model 320 based on the second medical data 30, as input variables. That is, the third loss function may be a function for comparing the input of the first sub-neural network model 310 and the output of the second sub-neural network model 320 based on the input of the first sub-neural network model 310. For example, the third loss function may be represented by Equation 3 below:

$$L_3 = Mean\ squared\ error(x_{real}^A, x_{iden}^A) \qquad (3)$$

where $x_{real}^A$ may be the second medical data 20 and $x_{iden}^A$ may be the fifth medical data 60. Although the mean squared error function is described as the loss function in Equation 3, the present disclosure is not limited to the corresponding function.

The processor 110 may obtain a third loss by performing the operation of the third loss function using the second medical data 20 and the fifth medical data 60 as input variables. The processor 110 may train the second sub-neural network model 320 so that the third loss is reduced. That is, the processor 110 may reconstruct parameters of the second sub-neural network model 320 to lower the third loss so that the difference between the fifth medical data 60 and the second medical data 20 is minimized. For example, when the second sub-neural network model 320 receives electrocardiogram data negative for hyperkalemia, the processor 110 may reconstruct parameters of the generative neural network 322 of the second sub-neural network model 320 in a direction in which the third loss is reduced by repeatedly performing the operation of the third loss function. In this case, the reconstruction of parameters of the generative neural network 322 of the second sub-neural network model 320 may be understood as a process of training the generative neural network 322 of the second sub-neural network model 320 so that the second sub-neural network model 320 generates electrocardiogram data negative for hyperkalemia, which is the same as the input electrocardiogram data. In this case, since the feature extraction neural network 321 of the second sub-neural network model 320 corresponds to the feature extraction neural network 210 of the first neural network model 200, the training of the feature extraction neural network 321 of the second sub-neural network model 320 may not be performed in the training process using the third loss function. Through the training via the third loss function, the second sub-neural network model 320 may accurately identify the domain of data to be transformed. That is, the processor 110 may train the second sub-neural network model 320 so that the second sub-neural network model 320 can generate the same domain as the input of the first sub-neural network model 320 through the training via the third loss function.

Meanwhile, the loss function for the training of the second neural network model including the first sub-neural network model 310 and the second sub-neural network model 320 may be represented by the sum of the first loss function, the second loss function, and the third loss function. That is, the processor 110 may train the second neural network model in a direction in which the total sum of losses obtained as a result of the operation of the individual loss functions described above is reduced. Through this training, the second neural network model may generate a counter factor for input medical data as grounds for explaining the features of the medical data that the first neural network model 200 has interpreted to predict the medical data to be positive or negative for the disease.

As described above, when the second neural network model is trained to generate a counter factor for the input medical data, the prediction result of the first neural network model 200 for the medical data and the prediction result of the first neural network model 200 for the output of the second neural network model having received the medical data may be obtained as exact opposite results. For example, when the prediction result of the pre-trained first neural network model 200 for the medical data is negative, the prediction result of the pre-trained first neural network model 200 for the output of the second neural network model having received the medical data may be positive. Accordingly, when the input of the second neural network model and the output of the second neural network model are compared with each other, the features of the medical data based on which the first neural network model 200 has determined the medical data to be positive or negative for the disease may be easily explained. That is, the second neural network model may be effectively used to explain the data interpretation and decision-making process of the first neural network model 200.

FIG. 4 is a graph showing simulation results of a second neural network model trained according to an embodiment of the present disclosure.

In order to verify the second neural network model trained according to an embodiment of the present disclosure, in the electrocardiogram record of a patient classified as negative for hyperkalemia, nine negative samples were extracted by moving a window of two seconds at intervals of one second. Then, the nine negative samples were input to the trained second neural network model and transformed into positive samples. FIG. 4 shows the results of comparison of the samples. The three overlapping curve groups shown in FIG. 4 are a first curve group 81 corresponding to the individual negative samples, a second curve group 82 corresponding to the positive samples generated by the second neural network model, and a third curve group 83 corresponding to samples of patients who were actually diagnosed as positive for hyperkalemia.

When the first curve group 81 and the second curve group 82 are compared with each other, it can be seen that the QRS duration and PR interval of the positive samples generated by the second neural network model are 1.14 times and 1.64 times larger than those of the negative samples, respectively, on an average basis. In addition, it can be seen that the proportion of Tall T in the positive samples generated by the second neural network model is 45.34%, which significantly increases more than twice that of the negative samples. In this case, Tall T may be understood as a case where the T amplitude is more than half the R peak amplitude. Furthermore, when the second curve group 82 and the third curve group 83 are compared with each other, it can be seen that the second curve group 82 exhibits a similar change pattern as the third curve group 83.

Based on the above-described results, it can be seen that the second neural network model trained according to an embodiment of the present disclosure may appropriately transform and propose morphological features of the electrocardiogram that affect a positive or negative prediction for hyperkalemia. That is, when the second neural network model trained according to an embodiment of the present disclosure is used, the criteria based on which a neural network model for diagnosing disease (e.g. the first neural network model of the present disclosure) predicts an electrocardiogram to be positive or negative for hyperkalemia by taking into consideration morphological features of the electrocardiogram may be explained.

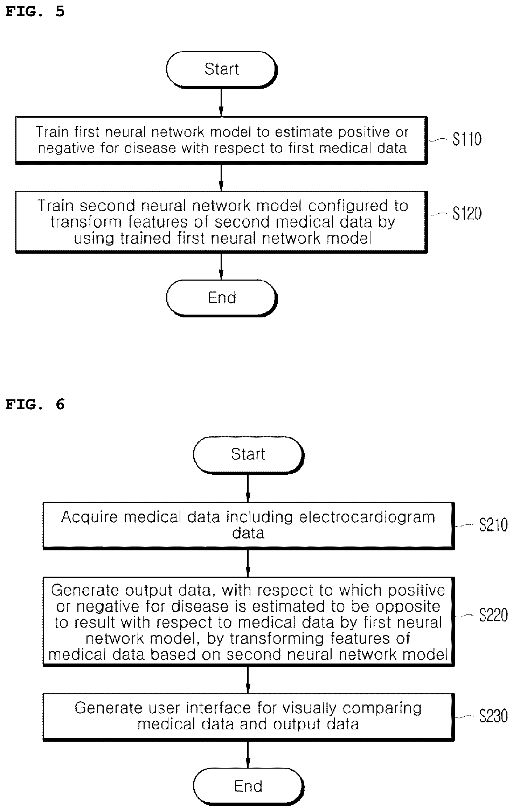

FIG. 5 is a flowchart showing a method of training a neural network model according to an embodiment of the present disclosure.

Referring to FIG. 5, the computing device 100 according to an embodiment of the present disclosure may train the first neural network model to estimate the first medical data to be positive or negative for a disease based on the first medical data in step S110. In this case, the first medical data may be biosignal-related data including sequential information such as an electrocardiogram. Furthermore, the disease for which the estimation is performed by the first neural network model may be hyperkalemia. Since hyperkalemia is frequently asymptomatic to an extremely dangerous level, it is difficult to diagnose hyperkalemia, especially outside a hospital. Accordingly, a method of diagnosing hyperkalemia using electrocardiograms, which are non-invasive and can be obtained in daily life, is attracting attention. According to this necessity, the first neural network model may be a model that receives electrocardiogram data and classifies whether hyperkalemia has occurred for the subject of measurement of the electrocardiogram data. However, since the types of data and disease are only examples, the task of the first neural network model of the present disclosure is not limited to the above-described example.

The computing device 100 may train the second neural network model configured to transform features of the second medical data so that a positive or negative for the disease with respect to the second medical data is estimated to be opposite by using the first neural network model, trained through step S110, in step S120. In this case, the second neural network model may be understood as a model that proposes grounds for explaining the determination and decision-making process of the first neural network model. Like the first medical data, the second medical data may be biosignal-related data, including sequential information, such as an electrocardiogram. Furthermore, the disease with respect to the second medical data may be hyperkalemia.

More specifically, the computing device 100 may transform features of the second medical data by using the second neural network model. The computing device 100 may estimate whether the second medical data whose features have been transformed is positive or negative for the disease by using the first neural network model trained through step S110. In this case, the second neural network model may include the first sub-neural network model configured to generate the third medical data by transforming features of the second medical data so that a positive or negative for the disease is estimated to be opposite to a result for the second medical data, and the second sub-neural network model configured to generate the fourth medical data by transforming features of the third medical data so that a positive or negative for the disease is estimated to be the same as a result for the second medical data.

For example, the computing device 100 may generate the third medical data, estimated to be positive, which is opposite to a result for the second medical data, by transforming features of the second medical data, estimated to be negative, based on the first sub-neural network model included in the second neural network model. When the second medical data is electrocardiogram data of a patient who has been diagnosed as negative for hyperkalemia, the computing device 100 may generate the third electrocardiogram data, which is electrocardiogram data estimated to be positive for hyperkalemia by the first neural network model, by transforming morphological features of the second medical data through the first sub-neural network model included in the second neural network model. That is, the first sub-neural network model may perform an operation of transforming features of data based on the morphological differences between electrocardiogram data negative for hyperkalemia and electrocardiogram data positive for hyperkalemia.

The computing device 100 may generate the fourth medical data corresponding to the second medical data estimated to be negative by transforming features of the third medical data based on the second sub-neural network model included in the second neural network model. When the third medical data is electrocardiogram data that is estimated to be positive for hyperkalemia by the first neural network model, the computing device 100 may generate the fourth medical data corresponding to the second medical data by transforming morphological features of the third medical data through the second sub-neural network model included in the second neural network model. That is, the second sub-neural network model may perform an operation of transforming features of data based on the morphological differences between electrocardiogram data negative for hyperkalemia and electrocardiogram data positive for hyperkalemia.

The computing device 100 may generate prediction data indicating a result of estimating whether the third medical data is positive or negative for the disease by inputting the third medical data to the first neural network model trained through step S110. When the second medical data is electrocardiogram data of a patient who has been diagnosed as negative for hyperkalemia, the third medical data generated by the first sub-neural network model needs to be estimated to be positive for hyperkalemia by the first neural network model. Accordingly, in order to evaluate whether the first sub-neural network model has appropriately generated the third medical data, the computing device 100 may determine whether the third medical data is data positive for hyperkalemia by analyzing the third medical data through the first neural network model.

The computing device 100 may generate the fifth medical data corresponding to the second medical data estimated to be negative by inputting the second medical data, estimated to be negative, to the second sub-neural network model. When the second medical data is electrocardiogram data of a patient who has been diagnosed as negative for hyperkalemia, the fifth medical data generated by the second sub-neural network model needs to be estimated to be also negative for hyperkalemia by the first neural network model like the second medical data. Accordingly, in order to evaluate whether the second sub-neural network model appropriately identifies the domain of data, the computing device 100 may determine whether the second sub-neural network model generates data that is the same as the second medical data.

Furthermore, the computing device 100 may train the neural network model based on a loss function that uses at least one of the second medical data whose features have been transformed and a positive or negative estimation result for the second medical data whose features have been transformed as an input variable. In other words, the computing device 100 may train the first sub-neural network model and second sub-neural network model included in the second neural network model based on a loss function that uses at least one of the fourth medical data, the fifth medical data, or a prediction data as an input variable. In this case, the loss function may include a first loss function for reflecting whether the first sub-neural network model generates an output corresponding to a positive for the disease by transforming features of an input corresponding to a negative for the disease, a second loss function for reflecting whether the second sub-neural network model restores the input of the first sub-neural network model by transforming features of the output of the first sub-neural network model, and a third loss function for reflecting whether the second sub-neural network model generates an output, which is the same as the input of the first sub-neural network model, based on the input of the first sub-neural network model.

For example, the computing device 100 may perform the operation of the first loss function using prediction data as an input variable, and may train the first sub-neural network model so that the first loss that is the result of the operation of the first loss function is reduced. When the second medical data is electrocardiogram data of a patient who has been diagnosed as negative for hyperkalemia, the computing device 100 may compare prediction data and ground truth (GT) corresponding to electrocardiogram data positive for hyperkalemia through the first loss function. The computing device 100 may update neural network parameters of the first sub-neural network model in a direction in which the first loss obtained as a result of the comparison is minimized. In this case, minimizing the first loss may be understood as training the first sub-neural network model so that the first neural network model can recognize the third medical data, generated by the first sub-neural network model, as electrocardiogram data of a patient who has been diagnosed as positive for true hyperkalemia.

The computing device 100 may perform the operation of the second loss function using the fourth medical data as an input variable, and may train at least one of the first sub-neural network model or the second sub-neural network model so that the second loss that is the result of the operation of the second loss function is reduced. When the second medical data is electrocardiogram data of a patient who has been diagnosed as negative for hyperkalemia, the computing device 100 may compare the second medical data and the fourth medical data through the first loss function. The computing device 100 may update neural network parameters of at least one of the first sub-neural network model or the second sub-neural network model in a direction in which the second loss obtained as a result of the comparison is minimized. In this case, minimizing the second loss may be understood as training at least one of the first sub-neural network model or the second sub-neural network model so that the fourth medical data generated by the second sub-neural network model corresponds to the data obtained by restoring the second medical data from the third medical data.

The computing device 100 may perform the operation of the third loss function using the fifth medical data as an input variable, and may train the second sub-neural network model so that the third loss that is a result of the operation of the third loss function is reduced. When the second medical data is electrocardiogram data of a patient who has been diagnosed as negative for hyperkalemia, the computing device 100 may compare the second medical data and the fifth medical data through the third loss function. The computing device 100 may update neural network parameters of the second sub-neural network model in a direction in which the third loss obtained as a result of the comparison is minimized. In this case, minimizing the third loss may be understood as training the second sub-neural network model so that the second sub-neural network model can output the fifth medical data that is the same as the second medical data.

The second neural network model trained as described above has the advantage of not depending on the architecture of the first neural network model that is the subject of analysis. In other words, the second neural network model according to an embodiment of the present disclosure has the advantage of having the flexibility to interpret any model having any architecture as long as it is a model that classifies whether data is positive or negative for a disease. Additionally, the second neural network model according to an embodiment of the present disclosure has the advantage of not necessarily requiring paired data for training. Since there is no need to prepare two electrocardiograms from the same patient to analyze the morphological differences in electrocardiograms under two different conditions, data collection for the training of the second neural network model is bound to be easy. The reason for this is that the second neural network model according to an embodiment of the present disclosure can recognize the morphological differences between two groups in an unpaired data set and generate a counter factor for an input.

FIG. 6 is a flowchart showing a method of interpreting medical data by using a neural network model according to an embodiment of the present disclosure.

Referring to FIG. 6, the computing device 100 according to an embodiment of the present disclosure may acquire medical data including electrocardiogram data in step S210. For example, when the computing device 100 is a server or client of a cloud system, the computing device 100 may receive electrocardiogram data through communication with a database in a hospital environment or through communication with an electrocardiogram measurement device. When the computing device 100 is a database within a hospital environment, the computing device 100 may generate electrocardiogram data through communication with an electrocardiogram measurement device within the hospital environment. In contrast, when the computing device 100 is an electrocardiogram measurement device, the computing device 100 may generate electrocardiogram data by performing an electrocardiogram measurement process on its own.

The computing device 100 may generate output data in which a positive or negative for the disease is estimated to be opposite to a result for the medical data by transforming features of the medical data, obtained through step S210, based on the second neural network model in step S220. In this case, the second neural network model may be pre-trained using the first neural network model trained to estimate whether medical data is positive or negative for the disease. Since the process of training the second neural network model using the pre-trained first neural network model is the same as in the description given above with reference to FIG. 5, a detailed description thereof will be omitted below. For example, when the computing device 100 acquires electrocardiogram data that has been determined to be negative for hyperkalemia, the computing device 100 may transform morphological features of the electrocardiogram data by inputting the acquired electrocardiogram data to the second neural network model. In this case, in order for the data to be generated as output to be determined to be positive for hyperkalemia, the second neural network model may transform the form of the input electrocardiogram data based on indices such as the PR interval, QRS duration and T amplitude of the electrocardiogram. The computing device 100 may generate electrocardiogram data, which can be determined to be positive for hyperkalemia, through the feature transformation using the second neural network model. In this case, the electrocardiogram data corresponding to the output of the second neural network model may be electrocardiogram data that can be determined to be positive by the first neural network model that classifies data as positive or negative for hyperkalemia.

In step S230, the computing device 100 may generate a user interface for visually comparing the medical data acquired through step S110 and the output data of the second neural network model generated through step S120. The output data of the second neural network model generated through step S120 may exhibit a counter factor based on the medical data acquired through step S110 and a positive or negative for the disease. In other words, the output data of the second neural network model generated through step S120 may be grounds for explaining the determination process of the artificial intelligence for classifying data as positive or negative for the disease. Accordingly, in order for a user to easily understand the determination process of the artificial intelligence through comparison between the medical data acquired through step S110 and the output data of the second neural network model generated through step S120, the computing device 100 may generate a user interface for visually comparing the two pieces of data.

For example, the computing device 100 may generate the medical data acquired through step S110 and the output data of the second neural network model generated through step S120 graphically in the form of a graph, as shown in FIG. 4. In this case, the computing device 100 may implement the two pieces of data generated graphically in the form of a graph through one area of the user interface. In order to facilitate comparison between the two pieces of data, the computing device 100 may display graphics in the form of a graph in one area of the user interface in an overlapping manner. In this case, the computing device 100 may perform the task of matching scales for clear comparison between overlapping curves.

Meanwhile, the computing device 100 may share the user interface with a client and provide the graphics to a user through the client. The computing device 100 may implement a user interface via its own input/output unit and provide the graphics to the user.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method of interpreting medical data based on explainable artificial intelligence, the method being performed by a computing device including at least one processor, the method comprising:

   training a first neural network model to estimate a positive or negative for a disease with respect to first medical data based on the first medical data; and
   training a second neural network model configured to transform features of second medical data so that a positive or negative for the disease with respect to the second medical data is estimated to be opposite by using the trained first neural network model.

2. The method of claim 1, wherein the second neural network model comprises:

   a first sub-neural network model configured to generate third medical data by transforming features of the second medical data so that a positive or negative for the disease is estimated to be opposite to a result with respect to the second medical data; and
   a second sub-neural network model configured to generate fourth medical data by transforming features of the third medical data so that a positive or negative for the disease is estimated to be the same as a result with respect to the second medical data.

3. The method of claim 1, wherein training the second neural network model configured to transform the one or more features of the second medical data so that the positive or negative for the disease with respect to the second medical data is estimated to be opposite by using the trained first neural network model comprises:

transforming features of the second medical data by using the second neural network model, and estimating a positive or negative with respect to the second medical data whose features have been transformed by using the trained first neural network model; and

training the second neural network model based on a loss function that uses at least one of the second medical data whose features have been transformed or a positive or negative estimation result with respect to the second medical data whose features have been transformed as an input variable.

4. The method of claim 3, wherein transforming the features of the second medical data by using the second neural network model and estimating the positive or negative with respect to the second medical data whose features have been transformed by using the trained first neural network model comprises:

generating third medical data estimated to be positive, which is opposite to a result with respect to the second medical data by transforming features of the second medical data estimated to be negative based on a first sub-neural network model included in the second neural network model; and

generating fourth medical data corresponding to the second medical data estimated to be negative by transforming features of the third medical data based on a second sub-neural network model included in the second neural network model.

5. The method of claim 4, wherein transforming the features of the second medical data by using the second neural network model and estimating the positive or negative with respect to the second medical data whose features have been transformed by using the trained first neural network model further comprises:
generating prediction data indicative of a positive or negative estimation result for the disease with respect to the third medical data by inputting the third medical data to the trained first neural network model.

6. The method of claim 5, wherein transforming the features of the second medical data by using the second neural network model and estimating the positive or negative with respect to the second medical data whose features have been transformed by using the trained first neural network model further comprises:
generating fifth medical data corresponding to the second medical data estimated to be negative by inputting the second medical data estimated to be negative to the second sub-neural network model.

7. The method of claim 6, wherein the loss function comprises:

a first loss function for evaluating whether the first sub-neural network model generates an output corresponding to a positive for the disease by transforming features of an input corresponding to a negative for the disease;

a second loss function for evaluating whether the second sub-neural network model restores an input of the first sub-neural network model by transforming features of the output of the first sub-neural network model; and

a third loss function for evaluating whether the second sub-neural network model generates an output, which is the same as the input of the first sub-neural network model, based on the input of the first sub-neural network model.

8. The method of claim 6, wherein training the second neural network model based on the loss function that uses the at least one of the second medical data whose features have been transformed or the positive or negative estimation result with respect to the second medical data whose features have been transformed as the input variable comprises:
training the first sub-neural network model and the second sub-neural network model based on a loss function that uses at least one of the fourth medical data, the fifth medical data, or the prediction data as an input variable.

9. The method of claim 8, wherein training the first sub-neural network model and the second sub-neural network model based on the loss function that uses the at least one of the fourth medical data, the fifth medical data, or the prediction data as the input variable comprises:

performing an operation of the first loss function using the prediction data as an input variable, and training the first sub-neural network model so that a first loss that is a result of the operation of the first loss function is reduced;

performing an operation of the second loss function using the fourth medical data as an input variable, and training at least one of the first sub-neural network model or the second sub-neural network model so that the second loss that is a result of the operation of the second loss function is reduced; and

performing an operation of the third loss function using the fifth medical data as an input variable, and training the second sub-neural network model so that a third loss that is a result of the operation of the third loss function is reduced.

**10.** The method of claim 1, wherein:

the first medical data and the second medical data include electrocardiogram data; and
the disease includes hyperkalemia.

**11.** The method of claim 10, wherein the features of the second medical data are based on morphological differences between the electrocardiogram data negative for hyperkalemia and the electrocardiogram data positive for hyperkalemia.

**12.** A method of interpreting medical data based on explainable artificial intelligence, the method being performed by a computing device including at least one processor, the method comprising:

acquiring medical data including electrocardiogram data; and
generating output data, with respect to which a positive or negative for a disease is estimated to be opposite to a result with respect to the medical data by a first neural network model, by transforming features of the medical data based on a second neural network model;
wherein the second neural network model has been pre-trained using the first neural network model trained to estimate a positive or negative for the disease with respect to medical data.

**13.** The method of claim 12, further comprising generating a user interface for visually comparing the medical data and the output data.

**14.** A computer program stored in a computer-readable storage medium, the computer program performing operations for interpreting medical data based on explainable artificial intelligence when executed on one or more processors, wherein the operations include operations of:

training a first neural network model to estimate a positive or negative for a disease with respect to first medical data based on the first medical data; and
training a second neural network model configured to transform features of second medical data so that a positive or negative for the disease with respect to the second medical data is estimated to be opposite by using the trained first neural network model.

**15.** A computing device for interpreting medical data based on explainable artificial intelligence, the computing device comprising:

a processor including at least one core;
memory including program codes that are executable on the processor; and
a network unit configured to acquire medical data;
wherein the processor:

trains a first neural network model to estimate a positive or negative for a disease with respect to first medical data based on the first medical data; and
trains a second neural network model configured to transform features of second medical data so that a positive or negative for the disease with respect to the second medical data is estimated to be opposite by using the trained first neural network model.

**FIG. 1**

**FIG. 2**

**FIG. 3**

**FIG. 4**

**FIG. 5**

```
              ┌──────────┐
              │  Start   │
              └────┬─────┘
                   │
                   ▼
   ┌─────────────────────────────────────────────┐
   │ Train first neural network model to estimate │ ─ S110
   │ positive or negative for disease with        │
   │ respect to first medical data                │
   └────────────────────┬────────────────────────┘
                        │
                        ▼
   ┌─────────────────────────────────────────────┐
   │ Train second neural network model           │ ─ S120
   │ configured to transform features of second  │
   │ medical data by using trained first neural  │
   │ network model                               │
   └────────────────────┬────────────────────────┘
                        │
                        ▼
                  ┌──────────┐
                  │   End    │
                  └──────────┘
```

**FIG. 6**

```
              ┌──────────┐
              │  Start   │
              └────┬─────┘
                   │
                   ▼
   ┌─────────────────────────────────────────────┐
   │ Acquire medical data including              │ ─ S210
   │ electrocardiogram data                      │
   └────────────────────┬────────────────────────┘
                        │
                        ▼
   ┌─────────────────────────────────────────────┐
   │ Generate output data, with respect to which │ ─ S220
   │ positive or negative for disease is         │
   │ estimated to be opposite to result with     │
   │ respect to medical data by first neural     │
   │ network model, by transforming features of  │
   │ medical data based on second neural network │
   │ model                                       │
   └────────────────────┬────────────────────────┘
                        │
                        ▼
   ┌─────────────────────────────────────────────┐
   │ Generate user interface for visually         │ ─ S230
   │ comparing medical data and output data       │
   └────────────────────┬────────────────────────┘
                        │
                        ▼
                  ┌──────────┐
                  │   End    │
                  └──────────┘
```